(19) Europäisches Patentamt / European Patent Office / Office européen des brevets

(11) **EP 2 767 326 A1**

(12) **EUROPEAN PATENT APPLICATION**

(43) Date of publication:
20.08.2014 Bulletin 2014/34

(21) Application number: 13199850.2

(22) Date of filing: 30.12.2013

(51) Int Cl.:
$B01D\ 53/46$ (2006.01)     $B01D\ 53/52$ (2006.01)
$B01D\ 53/78$ (2006.01)     $B01D\ 53/84$ (2006.01)
$C12M\ 1/00$ (2006.01)     $C10L\ 3/10$ (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 18.02.2013 EP 13461505

(71) Applicant: Politechnika Lódzka
90-924 Lódz (PL)

(72) Inventors:
• Zieminski, Krzysztof
93-355 Lódz (PL)
• Rogacki, Grzegorz
92-539 Lódz (PL)

(74) Representative: Witek, Rafal
WTS Patent Attorneys
Witek, Sniezko & Partners
ul. Rudolfa Weigla 12
53-114 Wroclaw (PL)

(54) **A process of optimization of biological removal of sulphur species such as hydrogen sulfide from biogas with a filter bed made of plastic**

(57)     A method of optimizing the microbiological purification of biogas resulting from the anaerobic fermentation of organic waste comprising the increase of the amount of liquid retained on a filling constituting a filter bed in a biofilter, a biotrickling filter or bioscrubber, characterised in that the process is conducted on a biological filter bed consisting of a filtrating filling composed of cylindrically shaped polyethylene elements with porous or corrugated walls, with a porosity no lesser than 60%, a loading density no greater than 289 kg/m$^3$, with a sprinkling intensity $Q_L$ not exceeding 0.5 *10$^4$ m$^3$/s.

**Fig. 1**

**Description**

The subject of the present invention is a method of optimizing the microbiological purification of biogas from the anaerobic fermentation of organic waste. The present invention belongs to the area of biotechnological methods of purifying biogas from various sources.

The biological purification of waste gas is based on two main processes, absorption into an aqueous phase and the biological degradation of resulting compounds. Absorption is a mass transfer process between the aqueous and gaseous phases. The goal of this process is the removal of one of the components of the gaseous mixture through its dissolution in the liquid phase. The essential condition for the absorption into the liquid phase is solubility of gas components in the absorbing liquid. In the case of chemical absorption, the gas mixture component reacts with the liquid phase forming new compounds.

In order to transfer a particular contaminant mass from a gas into a liquid, it is necessary for molecules to pass through the layer adjacent to the phase boundary and through the phase boundary, i.e. through the interphase surface. The rate of absorption is increased through extending the interphase surface and increasing the rate of diffusion. During the absorption of a gaseous component into the liquid, the transfer of mass from one phase to another occurs solely through the interphase boundary surface, therefore, the structure, selection of appropriate filling and operational parameters of the apparatus wherein the absorption occurs must ensure the formation of the largest possible phase contact surface. The goal of the filling is the formation of the most preferable contact conditions for the gas and liquid.

The biological purification of biogas is conducted in biofilter-type installations, a biofilters with a hydrated layer called and bioscrubbers. These systems are composed of three phases: solid (filling), liquid and gas. Contaminants in gas are absorbed and transferred into the aqueous phase, and then the products of water-absorbed gas reactions undergo degradation by microbes. The effect of the interaction of both processes is such that as a result of absorption the gasses are purified, whereas due to the biological degradation of contaminants the absorbent is regenerated.

In biofilters on the solid material that forms the filter bed becomes covered with a biofilm composed of microbes taking part in the contaminant degradation process. The correct functioning of biofilters is largely dependent on the selection of filtration materials. The selection should take into account such characteristics as granularity, water retention ability, durability, specific surface area, gas flow resistance and microbial settlement. The organic materials are used as filter beds in biofilters include peat and wood bark and wood residue composts. The aforementioned materials are prone to biodegradation as a result of which, their properties and structure are altered during biofilter activity. Thus the durability of such filter materials is limited. Another drawback is unhomogeneity, acidification ($H_2SO_4$, HCl) and compaction of the filter material occurring with time.

In biotrickling filters usually used for biodegrading gaseous contaminants, the liquid phase (water with nutrient salts) flows in a thin film down the filling, wetting the biological layer. The contaminated gas flows counter-current in relation to the liquid. Contaminants absorbed into the liquid diffuse into the biological layer, where they are biodegraded. The absorption and regeneration of the contaminants take place in one device. Biotrickling filters generally make use of materials, which do not provide any nutrients the microorganisms developing therein. For this reason, appropriate media are used in this type of apparatus, which are designed to wet the filter bed and deliver principal nutrients to the microorganisms. The microorganisms are inoculated onto a solid and microbiologically neutral filling such as Raschig rings, active carbon, glass spheres, ceramics, plastics or other synthetic materials.

In bioscrubbers, Contaminants from waste gasses are absorbed into liquid in which microorganisms are dispersed, whereas the biological degradation of contaminants occurs in a chamber with suspended microorganisms. The absorption and regeneration of the liquid thus occurs in two different devices. This system consists of a column, in which gaseous contaminants are absorbed by a liquid which is then transferred into the bioreactor. The microbiological degradation of organic compounds contained in the liquid phase occurs in the bioreactor.

Each of the devices for the biological removal of contaminants from gasses should ensure the essential conditions for the transfer of mass through the gas-liquid boundary (contact surface, phase flow characteristics, contact time) as well as the maintenance of gas stream parameters and liquid phase properties such as pH, temperature and moisture that favor bacteria growth.

An important factor affecting the process of the biological purification of biogas is the type of filling used, since microorganisms participating in $H_2S$ removal from biogas form a biofilm thereon. The microorganism carrier should have a homogenous structure and a constant pores volume. Its appropriate moisture level is also important, because an insufficiently wetted filter bed retards microorganism growth. A good filtration material for a biological filter bed should have the following characteristics: large porosity, a large specific surface area in order to increase the absorption capacity, low gas flow resistance, high water retention, high durability and large microbial settlement density. The filling should also have a low loading density and a minimal pressure decrease. According to known prior art, there many solutions that facilitate the even distribution of medium flow or that posses a developed contact surface area. Typical fillings in absorbers are Raschig rings, Lessing rings, Pall rings, Bialecki rings, 1÷13 rings, K-rings, Intalox blocks, Berl blocks, or Tellerette-type filling. Bio-type fillings include soil, peat, compost, wood bark and residues, active carbon, mineral wool, basalt, as well as ceramics, ground tire rubber or calcium alginate. Chung et al. (Chung, Y. C., C. Huang and C. P. Tseng. 1996. Operation optimization of Thiobacillus thioparus CH11 biofilter for hydrogen sulfide removal. Journal of Biotechnology 52: 31-38) discloses the use of calcium alginate as a filter bed in a biofilter. In the research by Gabriel and Deshusses (GABRIEL D., DESHUSSES A. M., 2003, Retrofitting existing chemical scrubbers to biotrickling filters for H2S emission control, in: Proceedings of the National Academy of Science of the United States of America, 100 (11), pp. 6308-6312), the removal of reduced sulphur compounds made use of a biotrickling filter filled with a polyurethane foam, which had been inoculated with *Thiobacillus sp.* Shareefdeen et al. (Shareefdeen, Z., B. Herner and S. Wilson. 2002. Biofiltration of nuisance sulfur gaseous odors from a meat rendering plant. Journal of Chemical Technology and Biotechnology 77: 1296-1299.) discloses a hydrogen sulphide removal process from biogas using a biofilter filled with wood fines (a commercial biofilter - BIOMIX™).

Because the selection of a filling for the biological filter bed is a significant factor influencing the biological purification of biogas, there is an extant need in the state of the art to provide a filling with appropriate parameters, which will enhance the process of removing contaminants and will make it possible to produce a biogas of high purity in terms of hydrogen sulphide, to a proportion of at least 1150-2000 mg/10 kWh of energy produced.

Unexpectedly, this has been achieved by the present invention.

The subject of present invention is a method of optimizing the microbiological degradation of biogas formed during the anaerobic fermentation of organic waste, comprising increasing the amount of liquid retention on filling forming the biological bed on the biofilter, biotrickling filter or bioscrubber, characterised in that the process is conducted on biological bed formed from a filtrating filling composed of cylindrically shaped polyethylene elements with porous and corrugated walls, with a porosity no less than 60%, with a loading density of no more than 289 kg/m$^3$, with a $Q_L$ sprinkling intensity of no more than 0.5 *10$^4$ m$^3$/s.

Preferably, the sprinkling intensity $Q_L$ is 0.2 *10$^4$ m$^3$/s.

Preferably, the biological filter bed is loaded with hydrogen sulphide up to 3772.2 H$_2$S/dm$^3$h.

The present invention is described more closely in the example embodiment in the drawing, wherein Fig. 1 shows a scheme of the experimental installation setup. Fig. 2 shows the dependence of total liquid retention ß [m$^3$/ m$^3$] on sprinkling intensity $Q_L$ [m$^3$/s]. Fig. 3 shows the dependence of hydrogen sulphide reduction (RE) on the filter bed loading with hydrogen sulphide (IL) in connection with the quantity of hydrogen sulphide removed over time (EC). The bed was made of plastic. Fig. 4 shows dependence of hydrogen sulphide reduction (RE) on the filter bed loading with hydrogen sulphide (IL) in connection with the quantity of hydrogen sulphide removed over time (EC). The filter bed consisted of Raschig rings.

**Example 1**

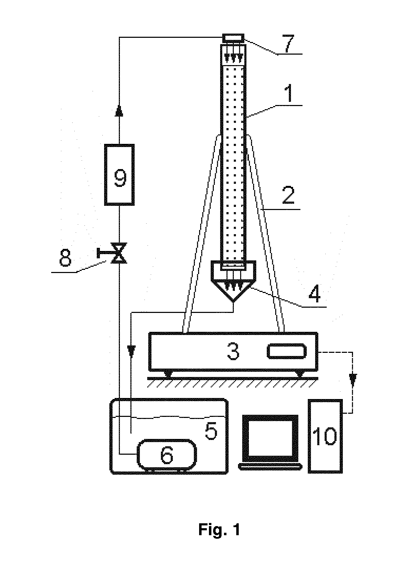

A scheme of the experimental setup is shown in Fig. 1. The column (1) was built using a pipe with a diameter of 110×106 mm and one metre long (1070 mm). The bottom of the column was fitted with a mesh bottom. The column loaded with the filling was weighed on a scale (3) with a weighing accuracy of 1/0.1 g. The water receptacle (4), without physical contact with the column, diverted the liquid into a recirculation tank (5) with a volume of 0.03 m$^3$. Water from the tank was supplied via a pump (6) back to the top of the column to the distributor (7). Due to the use of the recirculation tank, the water maintained a constant temperature of 22°C. The sprinkler at the top of the column also did not have any physical contact with the column. The sprinkling intensity was regulated with a valve (8) and measured using a system (9). Measurements were recorded using a computerized system (10).

The studies were performed using a ceramic filling (Rashig rings) and polyethylene shapes.

*1.a Determination of the loading density of the filling*

To determine the loading density, a previously weighed beaker with a nominal capacity of 3000 ml was loaded with a given filling to the bar denoting exactly 3000 ml, and the loaded beaker was reweighed. The weight of the beaker was subtracted from the combined weight of the filling and beaker. The resulting value was divided by 3 (litres) yielding the loading density expressed in g/litre = kg/m$^3$. The plastic filling had a low loading density (179 kg/m$^3$) in comparison to the ceramic Raschig rings (1033 kg/m$^3$).

*1.b Measurement of the porosity of the filling*

The measurement of the porosity of the filling was based on measuring 3000 ml of the filling in a beaker and pouring water onto it from another vessel with a known volume. The water was poured to a level identical to the filling and based on the decrease of the water level in the second vessel, the volume needed to filling the interparticle voids was calculated. Next, this volume was divided by the total filter bed volume, yielding the porosity, $\varepsilon$ [m$^3$/ m$^3$].

$$[\text{m}^3/\text{m}^3]$$

The plastic shapes had a higher porosity value (79.3%) in relation to the ceramic Raschig rings (55%).

*1.c Filling liquid retention calculations*

The column was loaded with a dry filling and weighed. Next, a particular sprinkling intensity was set and the weighing scale indications until a set state was achieved were recorded.

The total liquid retention measurement 13 [$\text{m}^3/\text{m}^3$] was made for the filling in the form of plastic shapes as well as total liquid retention 13 [$\text{m}^3/\text{m}^3$] in process conducted on Raschig rings.

To calculate the total liquid retention (ß) on the filling, as per a particular sprinkling intensity ($Q_L$), the weight of the column with dry filling ($W_{kws}$) was subtracted from the weight of the column with wet filling ($W_{kwl}$). The resulting difference was divided by the density of water ($\rho_L$) at room temperature (22°C) and the volume occupied by the filling. H denotes the height of the filling in the column. ($D_k$) denotes the internal diameter of the column.

$$\beta = \frac{W_{kwl} - W_{kws}}{\rho_L D_k H} \quad [\text{m}^3/\text{m}^3]$$

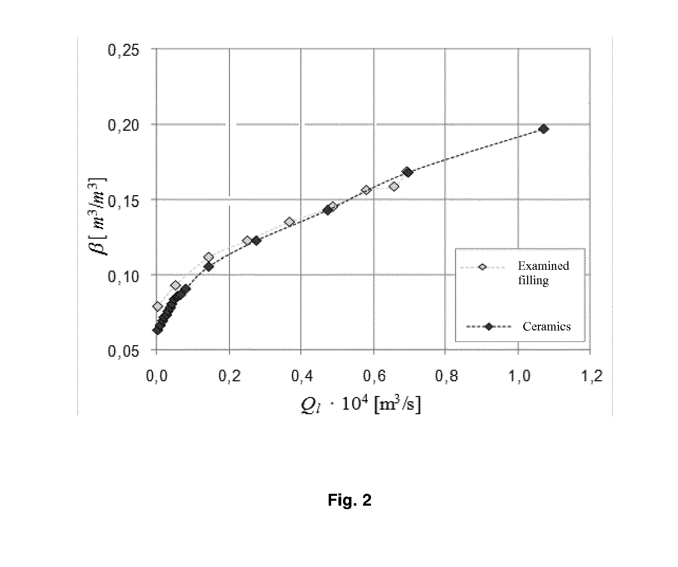

The results obtained were plotted in a (ß) over ($Q_L$) system shown in Fig. 2, yielding a dependence curve.

From the data shown in Fig. 2 it stems that the use of a filling in the study with a sprinkling intensity in the range of 0.5 $\text{m}^3$ * $10^4$ $\text{m}^3/\text{s}$ was characterised by a higher liquid retention in comparison to Raschig rings, commonly used in bioscrubbers and sprinkled filter beds. In the sprinkling intensity range of 0.004 to $0.47*10^4$ [$\text{m}^3/\text{s}$] liquid retention on the plastic filling was 5% greater than on Raschig rings. From the graphs in Fig. 2 it is evident that for the total retention of 1 $\text{m}^3$ of water on 1 $\text{m}^3$ of filling (ß) filling grows with the increase of sprinkling intensity in the column ($Q_L$). The most dynamic growth of total liquid retention (ß) for studied fillings can be observed in the range of sprinkling intensity up to $0.2*10^4$ $\text{m}^3/\text{s}$, particularly in the range from 0.004 to $0.2*10^4$ [$\text{m}^3/\text{s}$]. After going beyond the value of $0.6*10^4$ [$\text{m}^3/\text{s}$], however, the total liquid retention on the ceramic was greater than on the plastic filling.

**Example 2**

The experimental results demonstrating the functioning of a filter bed composed of a plastic material and Raschig rings and their effect on the removal of hydrogen sulphide from biogas were shown.

The proper selection of the filter bed and sprinkling intensity increases the total liquid retention on the bed. Increased liquid retention is beneficial to the trans-phase gas-liquid transfer and with that the growth of microorganisms, and thus the degree of hydrogen sulphide removal from biogas.

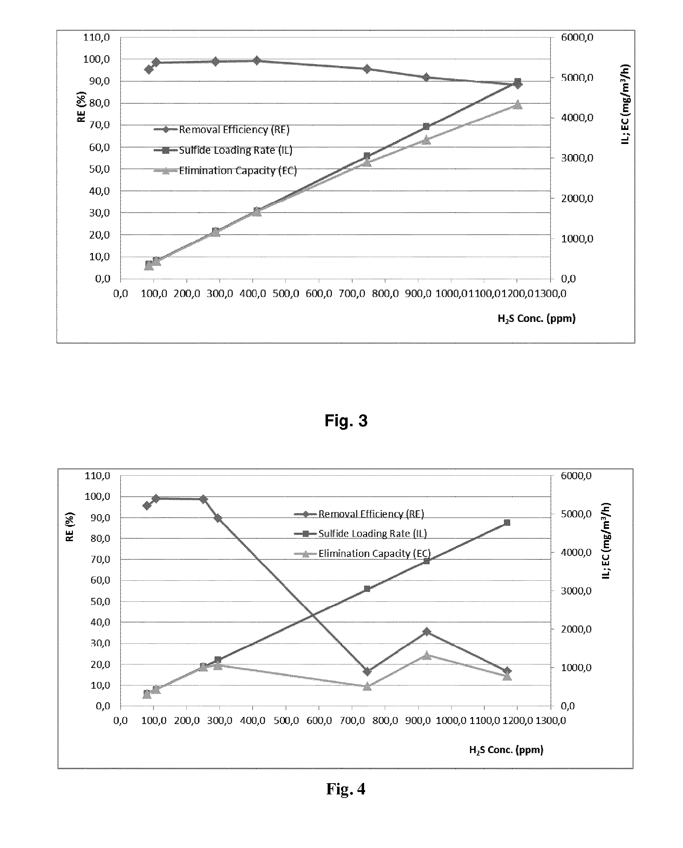

Fig. 3 shows the dependence of the reduction of hydrogen sulphide (RE) on filter bed loading with hydrogen sulphide (IL) in connection to the amount of hydrogen sulphide removed over time (EC). The bed was made of plastic.

Loading a plastic filter bed with a hydrogen sulphide load of up to 3772.7 mg $H_2S/\text{dm}^3$ h yields a high (over 90%) hydrogen sulphide reduction. Increasing the loading to 4899.2 mg $H_2S/\text{dm}^3$ h causes a slight decrease in the desulphuring effects.

This dependence is confirmed by the curve showing the amount of hydrogen sulphide removed [EC].

Up to a hydrogen sulphide loading level of 1684 mg $H_2S/\text{dm}^3$h the hydrogen sulphide removal curve is directly proportional to the amount of hydrogen sulphide loaded onto the bed. This results in the high observed (over 99%) hydrogen sulphide reduction.

Exceeding the aforementioned loading value, the amount of hydrogen sulphide loaded onto the bed was greater than the ability of the microbes to assimilate it and thus there was a decrease of desulphuring effects, but still the reduction rate was at around 90%.

Amount of hydrogen sulphide removed over time (Elimination Capacity, EC) 3

$$EC = Q/VB.(Cin-Cout) \ [\text{mg/m}^3/\text{h}]$$

where Q is the sprinkling intensity [$\text{m}^3/\text{h}$], VB is the filter bed volume ($\text{m}^3$), and Cin and Cout are $H_2S$ concentrations at the input and output of the filter bed, [$\text{mg/m}^3$].

Hydrogen sulphide reduction (Removal Efficiency, RE)

$$RE = (Cin - Cout)/ Cin . 100 [\%]$$

Cin and Cout are $H_2S$ concentrations at the input and output of the filter bed [$mg/m^3$ lub ppm].
Sulfide Loading Rate (IL)

$$IL = Q/VB . Cin [mg \ H_2S/m^3/h]$$

where Q is the sprinkling intensity [$m^3/h$], VB is the filter bed volume [$m^3$], Cin i Cout are $H_2S$ concentrations at the input and output of the filter bed [$mg/m^3$].

|  | $H_2S_{In}$ [ppm] | $H_2S_{Out}$ [ppm] | RE [%] | EC [$mg/m^3/h$] | IL [$mg/m^3/h$] |
|---|---|---|---|---|---|
| 1 | 86,7 | 4,2 | 95,3 | 336,1 | 353,1 |
| 2 | 108,8 | 1,7 | 98,5 | 436,6 | 443,4 |
| 3 | 288,8 | 3,5 | 98,9 | 1162,5 | 1176,7 |
| 4 | 413,3 | 3,0 | 99,2 | 1671,8 | 1684,0 |
| 5 | 747,0 | 38,0 | 95,5 | 2888,6 | 3043,4 |
| 6 | 926,0 | 77,7 | 91,7 | 3456,2 | 3772,7 |
| 7 | 1202,5 | 140,8 | 88,3 | 4325,4 | 4899,2 |

Fig. 4 Shows the dependence of hydrogen sulphide reduction (RE) on the loading rate of the filter bed with hydrogen sulphide (IL) in relation to the amount of hydrogen sulphide removed over time (EC). The bed was composed of Raschig rings.
A high degree of reduction, over 98%, was attained only with a hydrogen sulphide loading rate of up to 1027 mg $H_2S/dm^3h$. A further increase in the hydrogen sulphide load caused a systematic decrease in the desulphuring effects. A loading rate of 4762 mg $H_2S/dm^3h$ resulted in solely a 16.6% hydrogen sulphide reduction, whereas an a analogous load on a synthetic filter bed yielded an 88 % hydrogen sulphide reduction.

|  | $H_2S_{In}$ [ppm] | $H_2S_{Out}$ [ppm] | RE [%] | EC [$mg/m^3/h$] | IL [$mg/m^3/h$] |
|---|---|---|---|---|---|
| 1 | 80,3 | 3,7 | 95,5 | 312,4 | 327,3 |
| 2 | 108,8 | 1,2 | 98,9 | 438,6 | 443,4 |
| 3 | 252,2 | 3,5 | 98,6 | 1013,1 | 1027,4 |
| 4 | 294,5 | 32,5 | 89,7 | 1067,4 | 1199,8 |
| 5 | 747,0 | 619,8 | 16,4 | 518,1 | 3043,4 |
| 6 | 926,0 | 599,0 | 35,5 | 1332,2 | 3772,7 |
| 7 | 1169,0 | 977,2 | 16,6 | 781,6 | 4762,7 |

**Claims**

1.   A method of optimizing the microbiological purification of biogas resulting from the anaerobic fermentation of organic waste, comprising the increase of the amount of liquid retained on a filling constituting a filter bed in a biofilter, a biotrickling filter or bioscrubber, **characterised in that** the process is conducted on a biological filter bed formed from a filtrating filling composed of cylindricallyshaped polyethylene elements with porous or corrugated walls, with a porosity no lesser than 60%, a loading density no greater than 289 kg/$m^3$, with a sprinkling intensity $Q_L$ not exceeding 0.5 *$10^4$ $m^3/s$.

2.   A method according to Claim 1, **characterised in that** the sprinkling intensity ($Q_L$) is 0.2 * $10^4$ [$m^3/s$].

3.   A method according to Claim 1, **characterised in that** the filter bed is loaded with hydrogen sulphide at a rate of up to 3772,7 $H_2S/dm^3h$.

**Fig. 1**

**Fig. 2**

**Fig. 3**

Fig. 4

Europäisches
Patentamt

European
Patent Office

Office européen
des brevets

## EUROPEAN SEARCH REPORT

Application Number

EP 13 19 9850

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/084699 A1 (TOSHIBA KK [JP]; NAGAMORI YASUHIKO [JP]; OBARA TAKUMI [JP]; ASHIKAGA N) 9 July 2009 (2009-07-09) * page 18, line 26 - page 19, line 24 * * page 19, line 25 - page 20, line 4 * ----- | 1-3 | INV. B01D53/46 B01D53/52 B01D53/78 B01D53/84 C12M1/00 C10L3/10 |
| A | WO 2008/095057 A2 (NOVOZYMES AS [DK]; BORCHERT MARTIN [DK]; SAUNDERS PARIA [US]) 7 August 2008 (2008-08-07) * "In summary..."; page 15 * ----- | 1-3 | |
| A | US 5 236 677 A (TORRES-CARDONA MARIO D [MX] ET AL) 17 August 1993 (1993-08-17) * claims 1, 11 * ----- | 1-3 | |
| A | US 2007/180802 A1 (PARKER RICHARD D [US] ET AL) 9 August 2007 (2007-08-09) * paragraphs [0035], [0036] * ----- | 1-3 | |
| A | US 5 766 938 A (HONGO KENJIRO [JP]) 16 June 1998 (1998-06-16) * column 1, line 54 - line 61 * ----- | 1-3 | TECHNICAL FIELDS SEARCHED (IPC) |
| A | DE 41 29 101 C1 (PROF. DR. PETER KUNZ) 8 April 1993 (1993-04-08) * column 2, line 28 - line 42 * ----- | 1-3 | B01D C12M C10L |

The present search report has been drawn up for all claims

| Place of search | Date of completion of the search | Examiner |
|---|---|---|
| Munich | 5 May 2014 | Pöhlmann, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 13 19 9850

This annex lists the patent family members relating to the patent documents cited in the above-mentioned European search report.
The members are as contained in the European Patent Office EDP file on
The European Patent Office is in no way liable for these particulars which are merely given for the purpose of information.

05-05-2014

| Patent document cited in search report | Publication date | Patent family member(s) | Publication date |
|---|---|---|---|
| WO 2009084699 A1 | 09-07-2009 | US 2010261266 A1<br>WO 2009084699 A1 | 14-10-2010<br>09-07-2009 |
| WO 2008095057 A2 | 07-08-2008 | AU 2008210428 A1<br>CA 2675047 A1<br>CN 101688209 A<br>EP 2126090 A2<br>JP 2010517533 A<br>US 2010047866 A1<br>US 2010297723 A1<br>US 2011104779 A1<br>US 2013203156 A1<br>WO 2008095057 A2 | 07-08-2008<br>07-08-2008<br>31-03-2010<br>02-12-2009<br>27-05-2010<br>25-02-2010<br>25-11-2010<br>05-05-2011<br>08-08-2013<br>07-08-2008 |
| US 5236677 A | 17-08-1993 | NONE | |
| US 2007180802 A1 | 09-08-2007 | NONE | |
| US 5766938 A | 16-06-1998 | JP 3025196 B2<br>JP H09299743 A<br>US 5766938 A | 27-03-2000<br>25-11-1997<br>16-06-1998 |
| DE 4129101 C1 | 08-04-1993 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **CHUNG, Y. C. ; C. HUANG ; C. P. TSENG.** Operation optimization of Thiobacillus thioparus CH11 biofilter for hydrogen sulfide removal. *Journal of Biotechnology,* 1996, vol. 52, 31-38
- **GABRIEL D. ; DESHUSSES A. M.** Retrofitting existing chemical scrubbers to biotrickling filters for H2S emission control. *Proceedings of the National Academy of Science of the United States of America,* 2003, vol. 100 (11), 6308-6312
- **SHAREEFDEEN, Z. ; B. HERNER ; S. WILSON.** Biofiltration of nuisance sulfur gaseous odors from a meat rendering plant. *Journal of Chemical Technology and Biotechnology,* 2002, vol. 77, 1296-1299